(19) **Europäisches Patentamt**

**European Patent Office**

**Office européen des brevets**

(11) **EP 1 034 790 A2**

(12) # EUROPEAN PATENT APPLICATION

(43) Date of publication:
**13.09.2000 Bulletin 2000/37**

(21) Application number: **00301695.3**

(22) Date of filing: **02.03.2000**

(51) Int Cl.[7]: **A61K 38/21**, A61P 31/18
// (A61K38/21, 31:7056),
(A61K38/21, 38:20),
(A61K38/21, 38:16),
(A61K38/21, 31:00)

(84) Designated Contracting States:
**AT BE CH CY DE DK ES FI FR GB GR IE IT LI LU
MC NL PT SE**
Designated Extension States:
**AL LT LV MK RO SI**

(30) Priority: **02.03.1999 US 260388
12.03.1999 US 268521
08.04.1999 US 288358
03.12.1999 US 454004**

(71) Applicant: **SCHERING CORPORATION
Kenilworth, New Jersey 07033-0530 (US)**

(72) Inventors:
• **Laughlin, Mark A.**
**Edison, New Jersey 08820 (US)**
• **Glue, Paul W.**
**Flemington, New Jersey 08822 (US)**
• **Stalgis, Carlos O.**
**Millington, New Jersey 07946 (US)**

(74) Representative: **Ritter, Stephen David et al**
**Mathys & Squire
100 Grays Inn Road
London WC1X 8AL (GB)**

(54) **HIV therapy**

(57)    The uses of pegylated interferon-alfa, alone, and in association with an anti-HIV-1 drug therapy, and ribavirin for the preparation of a medicament for treating treatment-naive as well as treatment-experienced adult and pediatric patients having HIV-1 infections as well as patients co-infected with HIV-1 and HCV involving comprising a therapeutically effective amount of pegylated interferon-alfa, e.g. , pegylated interferon alfa-2b as monotherapy or preferably in association with a therapeutically effective amount of at least one of ribavirin, IL-2, IL-12, pentafuside alone or in combination with a therapeutically effective amount of an anti-HIV-1 drug therapy, e.g., HAART are disclosed.

**EP 1 034 790 A2**

**Description**

## BACKGROUND OF THE INVENTION

The present invention relates to use of pegylated interferon-alfa for preparation of medicaments for treating patients having immunodeficiency virus type-1 ("HIV-1") infections by administering a therapeutically effective amount of pegylated interferon-alfa sufficient to lower detectable HIV-1-RNA levels.

A-M. Vandamme et al., Antiviral Chemistry & Chemotherapy, 9:187-203 (1998) disclose current clinical treatments of HIV-1 infections in man including at least triple drug combinations or so-called Highly Active Antiretroviral Therapy ("HAART") ; HAART involves various combinations of nucleoside reverse transcriptase inhibitors ("NRTI"), non-nucleoside reverse transcriptase inhibitors ("NNRTI") and HIV protease inhibitors ("PI"). In compliant drug-naive patients, HAART is effective in reducing mortality and progression of HIV-1 to AIDS. However, these multidrug therapies do not eliminate HIV-1 and long-term treatment usually results in multidrug resistance. Development of new drug therapies to provide better HIV-1 treatment remains a priority.

## SUMMARY OF THE INVENTION

The present invention provides the use of pegylated interferon-alfa for preparation of a medicament for the treatment of HIV-1 infections in patients

The present invention also provides the use of pegylated interferon-alfa for preparation of a medicament for the treatment of HIV-1 infections in patients which comprises a therapeutically effective amount of pegylated interferon-alfa sufficient to lower detectable HIV-1-RNA in such patients.

The preferred pegylated interferon is pegylated interferon-alfa-2b which may be administered in association with a therapeutically effective amount of at least one of ribavirin, IL-2, IL-12, and pentafuside alone or in combination with a therapeutically effective amount of an anti-HIV-1 drug therapy.

The present invention also provides the use of an anti-HIV-1 drug therapy and pegylated interferon-alfa for preparation of a medicament for treatment of HIV-1 infections in patients

The present invention also provides the use of an anti-HIV-1 drug therapy and pegylated interferon-alfa for preparation of a medicament for treatment of HIV-1 infections in patients which comprises a therapeutically effective amount of pegylated interferon-alfa and a therapeutically effective amount of an anti-HIV-1 drug therapy sufficient to lower detectable HIV-1-RNA in such patients

The preferred anti-HIV-1 drug therapy is HAART

In preferred embodiements, the present invention also provides the treatment of patients having HIV-1 infections in two treatment time periods: (1) a first treatment time period of about two to about four weeks, about 0.1 to about 9.0 micrograms per kilogram of pegylated interferon-alfa-2b or about 50 to about 500 micrograms of pegylated interferon alfa-2a in a single dose QW or divided doses BIW is administered, followed by (2) a second treatment time period sufficient to lower detectable HIV-1 RNA levels, a therapeutically effective amount of an anti-HIV-1 therapy, e. g. HAART, in association with about 0.1 to about 9.0 micrograms per kilogram of pegylated interferon-alfa-2b or about 50 to about 500 micrograms of pegylated interferon alfa-2a in a single dose QW or divided doses BIW are administered

The present invention is also concerns the preparation of medicaments for treatment of treatment-naive as well as treatment-experienced adult and pediatric patients having HIV-1 infections as well as such patients co-infected with HIV-1 and HCV.

## DETAILED DESCRIPTION

The present invention concerns preparation of medicaments for treatment of patients having HIV-1 infections wherein the treatment comprises a therapeutically effective amount of pegylated interferon-alfa as monotherapy or in association with a therapeutically effective amount of at least one of ribavirin, interleukin-2("IL-2"), interleukin-12(" IL-12"), and pentafuside alone or in combination with an anti-HIV-1 therapy, especially, HAART, in accordance with good clinical practice to minimize detectable HIV-1-RNA plasma levels. See for example A-M. Vandamme et al., in Antiviral Chemistry & Chemotherapy, 9:187-203 (1998) and "Drugs for HIV Infection" in The Medical Letter Vol. 39 (Issue 1015) December 5, 1997, pages 111-116.. The initiation of the HAART may occur before, after or concurrently with administering a therapeutically effective amount of pegylated interferon-alfa in accordance with the present invention. In an embodiment of the present invention, the method of treating patients having HIV-1 infections comprises two treatment time periods. In the first treatment time period, a therapeutically effective amount of pegylated interferon-alfa is administered for a first treatment time period sufficient to lower detectable HIV-1-RNA plasma levels, by $\geq 0.5 \log_{10}$, i.e., $\geq$.

$5 \times 10^1$ preferably by at least 0.65 $\log_{10}$, i.e.,$\geq 0.65 \times 10^1$ , more preferably by at least one $\log_{10}$, i.e.,$\geq 1 \times 10^1$, most preferably by at least two $\log_{10}$, i.e.,

$\geq 1 \times 10^2$ , lower than the initial HIV-1-RNA plasma level. In the second treatment time period, the method entails continuing the administration of a therapeutically effective amount of pegylated interferon-alfa in association with a therapeutically effective amount of HAART in accordance with good clinical practice to minimize detectable HIV-1-RNA plasma levels .

The term "anti-HIV-1 therapy" as used herein means the multi-drug therapies used in current clinical treatments of HIV-1 infections, including but not limited to the multi-drug therapies, e.g., the triple and quadruple drug therapies such as disclosed by A-M. Vandamme et al., in Antiviral Chemistry & Chemotherapy, 9:187-203 (1998) which describes the current clinical treatments of HIV-1 infections, including when to start multi-drug therapy and which drugs to combine. The triple drug therapy may include two nucleoside and nucleotide reverse transcriptase inhibitors ("NRTIs") and one protease inhibitor ("PI"), but there are many issues to be considered in the choice of the precise HAART for any patient. See for example, Tables 1 & 2 and Figure 2 in A-M. Vandamme et al., and "Drugs for HIV Infection", listed hereinabove.

The term "patients having HIV-1 infections" as used herein means any patient -including a pediatric patient- having HIV-1 infection and includes treatment-naive patients and treatment-experienced patients having the H IV-1 infection as well as treatment-naive patients and treatment-experienced patients co-infected with the HIV-1 and hepatitis C virus ("HCV").

The term "pediatric patient" as used herein means a patient below the age of 17, and normally includes those from birth to 16 years of age.

The term "treatment-naive patients" as used herein means patients having HIV-1 or co-infected with the HIV-1 and HCV who have never been treated with any anti-retroviral drugs, e.g., NRTI, NNRTI, PI or any interferon, including but not limited to interferon-alfa, or pegylated interferon alfa.

The term "treatment-experienced" patients as used herein means those patients having HIV-1 or co-infected with the HIV-1 and HCV who have initiated some form of anti HIV therapy including, but not limited to HAART or some form of anti-HCV therapy, including but not limited to interferon-alfa, pegylated interferon alfa or ribavirin.

The term "patients having hepatitis C infections" as used herein means any patient-including a pediatric patient- having hepatitis C and includes treatment-naive patients having hepatitis C infections and treatment-experienced patients having hepatitis C infections as well as those pediatric, treatment-naive and treatment-experienced patients having chronic hepatitis C infections.

These patients having hepatitis C include those who are infected with mutiple HCV genotypes including type 1 as well as those infected with,e.g., HCV genotypes 2, 3, 4, 5 and/or 6 and other possible HCV genotypes.

The term "treatment-naive patients having hepatitis C infections" as used herein means patients with hepatitis C who have never been treated with ribavirin or any interferon, including but not limited to interferon-alfa, or pegylated interferon alfa.

The term" treatment-experienced patients having hepatitis C infections" as used herein means patients with hepatitis C who have been treated with ribavirin or any interferon, including but not limited to interferon-alfa, or pegylated interferon alfa, including relapsers and non-responder.

The term "relapsers" as used herein means treatment-experienced patients with hepatitis C who have relapsed after initial response to previous treatment with interferon alone, or in combination with ribavirin.

The term "non-responders" as used herein means treatment-experienced patients with hepatitis C who have not responded to prior treatment with any interferon alone, or in combination with ribavirin.

When the pegylated interferon-alfa administered is a pegylated interferon alfa-2b, the therapeutically effective amount of pegylated interferon alfa-2b administered during the treatment in accordance with the present invention, including in first and second treatment time periods, is in the range of about 0.1 to 9.0 micrograms per kilogram of pegylated interferon alfa-2b administered per week, in single or divided doses, preferably once a week (QW) or twice a week(BIW), preferably in the range of about 0.1 to about 9.0 micrograms per kilogram of pegylated interferon alfa-2b administered once a week (QW) or in the range of about 0.05 to about 4.5 micrograms per kilogram of pegylated interferon alfa-2b administered twice a week(BIW), or is in the range of about 0.5 to about 3.0 micrograms per kilogram of pegylated interferon alfa-2b administered per week, preferably in the range of about 0.5 to about 3.0 micrograms per kilogram of pegylated interferon alfa-2b administered once a week (QW) or in the range of about 0.25 to about 1.5 micrograms per kilogram of pegylated interferon alfa-2b administered twice a week , or is in the range of about 0.75 to about 1.5 micrograms per kilogram of pegylated interferon alfa-2b administered per week, most preferably is in the range of about 0.75 to about 1.5 micrograms per kilogram of pegylated interferon alfa-2b administered once a week or about 0.375 to about 0.75 micrograms per kilogram of pegylated interferon alfa-2b administered twice a week.

When the pegylated interferon-alfa administered to pediatric patients is a pegylated interferon alfa-2b, the therapeutically effective amount of pegylated interferon alfa-2b administered during the treatment in accordance with the present invention, including in first and second treatment time periods is in the range of about 0.1 to 9.0 micrograms per kilogram of pegylated interferon alfa-2b administered per week, in single or divided doses, preferably once a week (QW) or twice a week(BIW), more preferably about 0.1 to about 9.0 micrograms per kilogram of pegylated interferon alfa-2b administered once a week (QW), or about 0.05 to about 4.5 micrograms per kilogram of pegylated interferon alfa-2b administered per week, in single or divided doses, preferably once a week (QW) or twice a week(BIW), more preferably about 0.05 to about 4.5 micrograms per kilogram of pegylated interferon alfa-2b administered once a week, or preferably about 0.75 to about 3.0 micrograms per kilogram of pegylated interferon alfa-2b administered in single or divided doses, preferably once a week (QW) or twice a week(BIW), more preferably about 0.75 to about 3.0 micrograms per kilogram of pegylated interferon alfa-2b administered once a week or about 0.375 to about 1.5 micrograms per kilogram of pegylated interferon alfa-2b administered twice a week, and most preferably about 2.25 to about 2.6 micrograms per kilogram of pegylated interferon alfa-2b administered once a week or about 1.125 to about 1.3 micrograms per kilogram of pegylated interferon alfa-2b administered twice a week(BIW). In a prefferred embodiment of the present invention, pediatric doses of about 0.75, about 1.5 and about 3.0 micrograms per kilogram of pegylated interferon alfa-2b are administered once a week

When the pegylated interferon-alfa administered is a pegylated interferon alfa-2a, the therapeutically effective amount of pegylated interferon alfa-2a administered during the treatment in accordance with the present invention, including in first and second treatment time periods, is in the range of about 50 micrograms to about 500 micrograms once a week("QW"), preferably about 200 micrograms to about 250 micrograms QW or the effective amount is in the range of about 50 micrograms to about 250 micrograms twice a week, preferably about 100 micrograms to about 125 micrograms twice a week.

When the pegylated interferon-alfa administered to a pediatric patient is a pegylated interferon alfa-2a, the therapeutically effective amount of pegylated interferon alfa-2a administered during the treatment in accordance with the present invention, including in first treatment time period is in the range of about 50 micrograms to about 500 micrograms once a week("QW"), preferably about 300 micrograms to about 375 micrograms QW or the therapeutically effective amount of pegylated interferon alfa-2a administered to a pediatric patient is in the range of about 50 micrograms to about 250 micrograms twice a week, preferably about 150 micrograms to about 190 micrograms once a week

Ribavirin is administered to the patient in association with pegylated interferon-alfa, that is, before, after or concurrently with the administration of the pegylated interferon alfa. The pegylated interferon-alfa dose is preferably administered during the same period of time that the patient receives doses of ribavirin. The amount of ribavirin administered concurrently with the pegylated interferon-alfa is from about 400 to about 1600 mg per day, preferrably about 600 to about 1200 mg/day or about 800 to about 1200 mg day and most preferably about 1000 to about 1200 mg/kg a day. The pegylated interferon-alfa dose is also preferably administered to the pediatric patient during the same period of time that such patient receives doses of ribavirin. The amount of ribavirin administered to the pediatric patient concurrently with the pegylated interferon-alfa is from about 8 to about 15 mg per kilogram per day, preferrably about 8, 12 or 15 mg per kilogram per day, in divided doses.

Pegylated interferon-alfa formulations are not effective when administered orally, so the preferred method of administering the pegylated interferon-alfa is parenterally, preferably by subcutaneous, IV, or IM, injection. Ribavirin may be administered orally in capsule, tablet or liquid form in association with the parenteral administration of pegylated interferon-alfa . Of course, other types of administration of both medicaments, as they become available are contemplated, such as by nasal spray, transdermally, by suppository, by sustained release dosage form, and by pulmonary inhalation. Any form of administration will work so long as the proper dosages are delivered without destroying the active ingredient.

The term "nucleoside and nucleotide reverse transcriptase inhibitors" ("NTRI" s) as used herein means nucleosides and nucleotides and analogues thereof that inhibit the activity of HIV-1 reverse transcriptase, the enzyme which catalyzes the conversion of viral genomic HIV-1 RNA into proviral HIV-1 DNA.

Typical suitable NRTIs include zidovudine (AZT) available under the RETROVIR tradename from Glaxo-Wellcome Inc., Research Triangle, NC 27709; didanosine (ddl) available under the VIDEX tradename from Bristol-Myers Squibb Co., Princeton, NJ 08543; zalcitabine (ddC) available under the HIVID tradename from Roche Pharmaceuticals, Nutley, NJ 07110; stavudine (d4T) available under the ZERIT trademark from Bristol-Myers Squibb Co., Princeton, NJ 08543; lamivudine (3TC) available under the EPIVIR tradename from Glaxo-Wellcome Research Triangle, NC 27709; abacavir (1592U89) disclosed in W096/30025 and available under the ZIAGEN tradename from Glaxo-Wellcome Research Triangle, NC 27709; adefovir dipivoxil [bis(POM)-PMEA] available under the PREVON tradename from Gilead Sciences, Foster City, CA 94404; lobucavir (BMS-180194), a nucleoside reverse transcriptase inhibitor disclosed in EP-0358154 and EP-0736533 and under development by Bristol-Myers Squibb, Princeton, NJ 08543; BCH-10652, a reverse transcriptse inhibitor (in the form of a racemic mixture of BCH-10618 and BCH-10619) under development by Biochem Pharma, Laval, Quebec H7V, 4A7, Canada; emitricitabine [(-)-FTC] licensed from Emory University under Emory Univ. U.S. Patent No. 5,814,639 and under development by Triangle Pharmaceuticals, Durham, NC 27707; beta-L-FD4(also called beta-L-D4C and named beta-L-2', 3'-dicleoxy-5-fluorocytidene) licensed by Yale University to Vion Pharmaceuticals, New Haven CT 06511; and DAPD, the purine nucleoside, (-)-beta-D-2,6,-diaminopurine dioxolane disclosed in EP 0656778 and licensed by Emory University and the University of Georgia to Triangle Pharmaceuticals, Durham, NC 27707; and lodenosine (FddA), 9-(2,3-dideoxy-2-fluoro-b-D-threo-pentofuranosyl)adenine, a acid stable purine-based reverse transcriptase inhibitor discovered by the NIH and under development by U.S. Bioscience Inc., West Conshohoken, PA. 19428.

The term "non-nucleoside reverse transcriptase inhibitors" ("NNRTI"s) as used herein means non-nucleosides that inhibit the activity of HIV-1 reverse transcriptase.

Typical suitable non-nucleoside reverse transcriptase inhibitors include nevirapine (BI-RG-587) available under the VIRAMUNE tradename from Boehringer Ingelheim, the manufacturer for Roxane Laboratories, Columbus, OH 43216; delaviradine (BHAP, U-90152) available under the RESCRIPTOR tradename from Pharmacia & Upjohn Co., Bridgewater NJ 08807; efavirenz (DMP-266) a benzoxazin-2-one disclosed in W094/03440 and available under the SUSTIVA tradename from DuPont Pharmaceutical Co., Wilmington, DE 19880-0723; PNU-142721, a furopyridine-thiopyrimide under development by Pharmacia and Upjohn, Bridgewater NJ 08807; AG-1549 (formerly Shionogi # S-1153); 5- (3,5-dichlorophenyl)- thio-4-isopropyl-1-(4-pyridyl)methyl-IH-imidazol-2-ylmethyl carbonate disclosed in WO 96 /10019 and under clinical development by Agouron Pharmaceuticals, Inc., LaJolla CA 92037-1020; MKC-442 1-(ethoxymethyl)-5-(1-methylethyl)-6-(phenylmethyl)-(2,4(1H,3H)-pyrimidinedione discovered by Mitsubishi Chemical Co. and under development by Triangle Pharmaceuticals, Durham, NC 27707; and (+)-calanolide A (NSC-675451) and B coumarin derivatives disclosed in NIH U.S. Patent No. 5,489,697, licensed to Med Chem Research, which is co-developing (+) calanolide A with Vita-Invest as an orally administrable product.

The term "protease inhibitor" ("PI") as used herein means inhibitors of the HIV-1 protease, an enzyme required for the proteolytic cleavage of viral polyprotein precursors (e.g., viral GAG and GAG Pol polyproteins), into the individual functional proteins found in infectious HIV-1. HIV protease inhibitors include compounds having a peptidomimetic structure, high molecular weight (7600 daltons) and substantial peptide character, e.g. CRIXIVAN(available from Merck) as well as nonpeptide protease inhibitors e.g., VIRACEPT (available from Agouron).

Typical suitable protease inhibitors include saquinavir (Ro 31-8959) available in hard gel capsules under the INVIRASE tradename and as soft gel capsules under the FORTOUASE tradename from Roche Pharmaceuticals, Nutley, NJ 07110-1199; ritonavir (ABT-538) available under the NORVIR tradename from Abbott Laboratories, Abbott Park, IL 60064; indinavir (MK-639) available under the CRIXIVAN tradename from Merck & Co., Inc., West Point, PA 19486-0004; nelfnavir (AG-1343) available under the VIRACEPT tradename from Agouron Pharmaceuticals, Inc., La-Jolla, CA 92037-1020; amprenavir (141W94), a non-peptide protease inhibitor under development by Vertex Pharmaceuticals, Inc., Cambridge, MA 02139-4211 and available from Glaxo-Wellcome, Research Triangle, NC under an expanded access program; lasinavir (BMS-234475) available from Bristol-Myers Squibb, Princeton, NJ 08543 (originally discovered by Novartis, Basel, Switzerland (CGP-61755); DMP-450, a cyclic urea discovered by Dupont and under development by Triangle Pharmaceuticals; BMS-2322623, an azapeptide under development by Bristol-Myers Squibb, Princeton, NJ 08543 as a 2nd-generation HIV-1 PI; and ABT-378 under development by Abbott , Abbott Park, IL 60064; and AG-1549 an orally active imidazole carbamate discovered by Shionogi (Shionogi #S-1153) and under development by Agouron Pharmaceuticals, Inc., LaJolla CA 92037-1020;

The term "anti-HIV-1 therapy" as used herein means any anti-HIV-1 drug found useful for treating HIV-1 infections in man alone, or as part of multidrug combination therapies, especially the triple and quadruple combination therapies called HAART.

Typical suitable anti-HIV-1 therapies include, but are not limited to multidrug combination therapies such as (i) at least three anti-HIV-1 drugs selected from two NRTIs, one PI, a second PI, and one NNRTI; and (ii) at least two anti-HIV-1 drugs selected from , NNRTIs and PIs ;see Talbes I, II and III, hereinafter.

Typical suitable HAART - multidrug combination therapies- include (a) triple combination therapies such as two NRTIs and one PI ; or (b) two NRTIs and one NNRTI ; and (c) quadruple combination therapies such as two NRTIs , one PI and a second PI or one NNRTI. In treatment-naive patients, it is preferred to start anti-HIV-1 treatment with the triple combination therapy; the use of two NRTIs and one PI is prefered unless there is intolerance to PIs. Drug compliance is essential. The CD4$^+$ and HIV-1-RNA plasma levels should be monitored every 3-6 months. Should viral load plateau, a fourth drug,e.g., one PI or one NNRTI could be added. See the Table A hereinbelow.

Table A

| ANTI-HIV-1 MULTI DRUG COMBINATION THERAPIES | |
|---|---|
| A. Triple Combination Therapies | |
| 1. | Two NRTIs[1]+ one PI[2] |
| 2. | Two NRTIs[1] + one NNRTI1[3] |
| B. Quadruple Combination Therapies[4] | |
| | Two NRTIs + one PI + a second PI or one NNRTI |
| C. ALTERNATIVES:[5] | |
| | Two NTRI1 |
| | One NTRI[5] + one PI[2] |
| | Two PIs[6] ± one NTRI[7] or NNRTI[3] |
| | One PI[2] + one NRTI[7] + one NNRTI[3] |

FOOTNOTES TO TABLE A 1. One of the following: zidovudine + lamivudine; zidovudine + didanosine; stavudine + lamivudine; stavudine + didanosine; zidovudine + zalcitabine; See also Table I

2. Indinavir, nelfinavir, ritonavir or saquinavir soft gel capsules. Ritonavir is used less frequently because of troublesome adverse effects. The old formulation of saquinavir was used least often because of its poor bioavailability and limited effectiveness, but the new saquinavir formulation should be more effective. See also Table III.

3. Nevirapine or delavirdine. See also Table II

4. See A-M. Vandamne et al Antiviral Chemistry + Chemotherapy 9:187 at p 193-197 and Figures 1 + 2.

5. Alternative regimens are for patients unable to take a recommended regimen because of compliance problems or toxicity, and for those who fail or relapse on a recommended regimen. Double nucleoside combinations may lead to HIV- resistance and clinical failure in many patients.

6. Most data obtained with saquinavir and ritonavir (each 400 mg bid).See also Table III

7. Zidovudine, stavudine or didanosine.See also Table I

Other anti-HIV-1 drugs useful for administration in association with pegylated interferon alfa include hydroxyurea, ribavirin, IL-2 and IL-12, and Yissum Project No. 11607 . These above-listed anti-HIV-1 drugs may also be administered in association with pegylated interferon alfa in association with any anti-HIV-1 drug therapy, especially the triple and quadruple drug combinations called HAART.

Hydroyurea (Droxia) is a ribonucleoside triphosphate reductase inhibitor, the enzyme involved in the activation of T-cells. Hydroxyurea discovered at the NCI is under development by Bristol-Myers Squibb. In preclinical studies, it was shown to have a synergistic effect on the activity of didanosine and has been studied with stavudine.

Yissum Project No. 11607, a synthetic protein based on the HIV -1 Vif protein under preclinical development by Yissum Research Development Co., Jerusalem 91042, Israel.

The pegylated inteferon alfa, $PEG_{12000}$ -IFN-alfa2b(available from Schering-Plough Research Institute, Kenilworth, NJ) increased the in vitro anti HIV-1 activity of ribavirin. The combination of $PEG_{12000}$-IFN-alfa2b and ribavirin inhibited HIV replication in vitro using phytohemagylutinin ("PHA" - P) - activated peripheral blood mononuclear cells ("PBMCs") at doses corresponding to plasmatic concentrations observed in animals and man. Healthy PBMCs were separated from a buffy-coat of one HIV-seronegative blood donor by Ficoll-Hypaque density gradient centrifugation. PBMCs were activated by 1 μg/ml phytohemagglutinin (PHA-P) for two days in cell culture medium A: RPMI 1640 supplemented with 10% heat-inactivated (+56°C, 45 min.) fetal calf plasma (FCS), 2 mM L-glutamine and a tri-antibiotic mixture (penicillin, streptomycin, neomycin; PSN). After these two days, cells were washed and cultured at one million cell per milliliter in cell culture medium B: cell culture medium A supplemented with 20 IU/ml recombinant human interleukin-2. Cells were maintained at +37°C in a 5% $CO_2$-air humidified atmosphere. Experiments were repeated twice with cells of other blood donors. In total, three independent experiments were performed.

PBMCs were infected with 1,000 50% Tissue Culture Infectious Doses (TCID50) of the reference HIV-1-LAI strain [F.Barre'-Sinoussi, Science, 1983, 220, 868-871]. This strain has been amplified using PHA-P-activated umbilical blood mononuclear cells (UBMC). Viral stock has been then titrated on PHA-P activated PBMC by end-point dilution. TCID50 was then calculated using Karber's formula [Arch. Exp. Path. Pharmak., 1931, 162, 126-133].

$PEG_{12000}$-IFN-α2b and ribavirin, alone and in combination, and AZT used as a control, were administrated 24 hours before HIV-1 infection and maintained all along the culture. Three doses of $PEG_{12000}$-IFN-α2b and ribavirin were used.

200,000 PHA-P-activated PBMCs were added to each well of 96-well microplates. Cells were 24 hour-pretreated prior to infection with the reference HIV-1-LAI strain. Twice a week, cell supernatants were collected, and drugs and medium were renewed. At day 7, RT activity were determined in cell supernatants, and potential cytotoxic effects of drugs and drug combinations were evaluated by microscopic observation.

Viral replication was measured by determining reverse transcriptase ("RT') activity in cell supernatants using Retro-Sys® kit, according to manufacturer's recommendations (Innovagen, Lund, Sweden).

Effective doses were calculated using cumulative RT activities with Chou J. and TC. microcomputer software.

The combined effects were analyzed using either the combination index (CI) [Chou & Talalay, 1984] with J and TC Chou microcomputer software, or the fractionary inhibitory concentration (FIC) index [Antimicrob. Agents. Chemother., 1987, 31, 1613-1617]. When the CI or FIC index is equal to 1, the combination is additive. When it is below 1.0, the combination is synergistic, and when it is above 1.0, the combination is judged as antagonistic.

$PEG_{12000}$-IFN-alfa2b as well as the combination of $PEG_{12000}$-IFN-alfa2b and ribavirin inhibited the HIV replication at doses corresponding to plasmatic concentrations measured in mice and HIV-1 infected patients [BE. Gilbert, et al. Antimicrob. Agents Chemother., 1988, 32. 117-121; E. Connor at al., Antimicrob. Agents Chemother., 1993, 37, 537-539].

IL-2 is disclosed in Ajinomoto EP-0142268 , Takeda EP-0176299, and Chiron U. S. Patent Nos. RE 33653, 4530787, 4569790, 4604377, 4748234, 4752585, and 4949314 is available under the PROLEUKIN(aldesleukin) tradename from Chiron Corp., Emeryville, CA 94608-2997 as a lyophilized powder for IV infusion or sc administration upon reconstitution and dilution with water; doses of about 1 to about 20 million IU/day, sc is preferred; a dose of about 15 million IU/day, sc is more preferred.

IL-12 is disclosed in WO96/25171 and is available from Roche Pharmaceuticals, Nutley, NJ 07110-1199 and American Home Prodocts, Madison, NJ 07940; a dose of about 0.5 microgram/kg/day to about 10 microgram/kg/day, sc.

Pentafuside (DP-178, T-20) a 36-amino acid synthetic peptide,disclosed in U.S. Patent No.5,464,933 licensed from Duke University to Trimeris which is developing pentafuside in collaboration with Duke University; pentafuside acts by inhibiting fusion of HIV-1 to target membranes. Pentafuside (3-100 mg /day) is given as a continuous sc infusion or injection together with efavirenz and 2 PI's to HIV-1 positive patients refractory to a triple combination therapy; use of 100 mg/day is preferred.

The term " interferon-alfa " as used herein means the family of highly homologous species-specific proteins that inhibit viral replication and cellular proliferation and modulate immune response. Typical suitable interferon-alfas include, but are not limited to, recombinant interferon alfa-2b such as Intron-A interferon available from Schering Corporation, Kenilworth, N.J., recombinant interferon alfa-2a such as Roferon interferon available from Hoffmann-La Roche, Nutley, N.J., recombinant interferon alpha-2C such as Berofor alpha 2 interferon available from Boehringer Ingelheim Pharmaceutical, Inc., Ridgefield, CT., interferon alpha-n1, a purified blend of natural alfa interferons such as Sumiferon available from Sumitomo, Japan or as Wellferon interferon alpha-n1 (INS) available from the Glaxo-Wellcome Ltd., London, Great Britain, or a consensus alpha interferon such as those described in U.S. Patent Nos. 4,897,471 and 4,695,623 (especially Examples 7, 8 or 9 thereof) and the specific product available from Amgen, Inc., Newbury Park, CA, or interferon alfa-n3 a mixture of natural alfa interferons made by Interferon Sciences and available from the Purdue Frederick Co., Norwalk, CT., under the Alferon Tradename. The use of interferon alfa-2a or alpha 2b is preferred. Since interferon alpha 2b, among all interferons, has the broadest approval throughout the world for treating chronic hepatitis C infection, it is most preferred. The manufacture of interferon-alpha 2b is described in U.S. Patent No. 4,530,901.

The term "pegylated interferon alfa" as used herein means polyethylene glycol modified conjugates of interferon alfa, preferably interferon alfa-2a and -2b. The preferred polyethylene-glycol-interferon alfa -2b conjugate is $PEG_{12000}$-interferon alfa 2b. The phrases "12,000 molecular weight polyethylene glycol conjugated interferon alpha" and "$PEG_{12000}$-IFN alfa" as used herein mean conjugates such as are prepared according to the methods of International Application No. WO 95/13090 and containing urethane linkages between the interferon alfa-2a or -2b amino groups and polyethylene glycol having an average molecular weight of 12000.

The preferred $PEGI_{12000}$-interferon alfa-2b is prepared by attaching a PEG polymer to the epsilon amino group of a lysine residue in the IFN alfa-2b molecule. A single $PEG_{12000}$ molecule is conjugated to free amino groups on an IFN alfa-2b molecule via a urethane linkage. This conjugate is characterized by the molecular weight of $PEG_{12000}$ attached. The PEG12000-IFN alfa-2b conjugate is formulated as a lyophilized powder for injection. The objective of conjugation of IFN alfa with PEG is to improve the delivery of the protein by significantly prolonging its plasma half-life, and thereby provide protracted activity of IFN alfa.

Other interferon alfa conjugates can be prepared by coupling an interferon alfa to a water-soluble polymer. A non-limiting list of such polymers include other polyalkylene oxide homopolymers such as polypropylene glycols, polyoxyethylenated polyols, copolymers thereof and block copolymers thereof. As an alternative to polyalkylene oxide-based polymers, effectively non-antigenic materials such as dextran, polyvinylpyrrolidones, polyacrylamides, polyvinyl alcohols, carbohydrate-based polymers and the like can be used. Such interferon alfa-polymer conjugates are described in U.S. Patent No. 4,766,106, U.S. Patent No. 4,917,888, European Patent Application No. 0 236 987, European Patent Application Nos. 0510 356, 0 593 868 and 0 809 996 (pegylated interferon alfa-2a) and International Publication No. WO 95/13090.

Pharmaceutical composition of pegylated interferon alfa-suitable for parenteral administration may be formulated with a suitable buffer, e.g., Tris-HCI, acetate or phosphate such as dibasic sodium phosphate/monobasic sodium phosphate buffer, and pharmaceutically acceptable excipients ( e.g., sucrose), carriers (e.g. human plasma albumin), toxicity agents (e.g. NaCI), preservatives (e.g. thimerosol, cresol or benylalcohol), and surfactants( e.g. tween or polysorabates) in sterile water for injection. The pegylated interferon alfa-may be stored as lyophilized powders under a refrigeration at 2°-8°C. The reconstituted aqueous solutions are stable when stored between 2° and 8°C and used within 24 hours of reconstitution. See for example U.S. Patent Nos, 4,492,537; 5,762,923 and 5,766,582.The reconstituted aqueous solutionsmay also be stored in prefilled, multi-dose syringes such as those useful for delivery of drugs such as insulin. Typical suitable syringes include systems comprising a prefilled vial attached to a pen-type syringe such as the NOVOLET Novo Pen available from Novo Nordisk, as well as prefilled, pen-type syringes which allow easy self-injection by the user. Other syringe systems include a pen-type syringe comprising a glass cartridge containing a diluent and lyophilized pegylated interferon alfa powder in a separate compartment.

A person suffering from chronic hepatitis C infection may exhibit one or more of the following signs or symptoms:

(a) elevated ALT,

(b) positive test for anti-HCV antibodies,

(c) presence of HCV as demonstrated by a positive test for the presence of HCV-RNA in the serum,

(d) clinical stigmata of chronic liver disease,

(e) hepatocelluar damage.

To practice the invention, the combination therapy of pegylated interferon-alfa and ribavirin is administered in association with anti-retroviral therapy,e.g., HAART, to the patient having HIV-1 infection and exhibiting one or more of the above signs or symptoms in the first and second treatment time periods in amounts sufficient to eliminate or at least alleviate one or more of the signs or symptoms., and to lower the HCV-RNA serum levels by at least a power of ten, and preferably to eradicate detectable HCV-RNA at least by the end of the second treatment time period and to maintain no detectable HCV-RNA for at least 24 weeks after the end of the second treatment time period. The sum of the first and second treatment time periods is about 40-50 weeks, and preferrably is 48 weeks. Administration of the ribavirin may be discontinued after the end of the second time period depending upon the judgment of the attending clinician.

The term " no detectable HCV-RNA" in the context of the present invention means that there are fewer than 100 copies of HCV-RNA per ml of serum of the patient as measured by quantitative, multi-cycle reverse transcriptase PCR methodology. HCV-RNA is preferably measured in the present invention by research-based RT-PCR methodology well known to the skilled clinician. This methodology is referred to herein as HCV-RNA/qPCR. The lower limit of detection of HCV-RNA is 100 copies/mL. Serum HCV-RNA/qPCR testing and HCV genotype testing will be performed by a central laboratory. See also J. G. McHutchinson et al. (N. Engl. J. Med., 1998, 339:1485-1492), and G. L. Davis et al. (N. Engl. J. Med. 339:1493-1499).

In a preferred embodiment of the present invention, those patients co-infected with HIV-1 and HCV infections are treated with pegylated interferon alfa in combination with ribavirin and a HAART combination considered appropriate by the attending clinician and the patient; use of the interferon alfa-2b-ribavirin combination therapy sold by Schering Corp. under the REBETRON tradename is preferred. See also J. G. McHutchinson et al. (N. Engl. J. Med., 1998, 339: 1485-1492), and G. L. Davis et al. (N. Engl. J. Med. 339:1493-1499). Ribavirin, 1-β-D-ribofuranosyl-1H-1,2,4-triazole-3-carboxamide, available from ICN Pharmaceuticals, Inc., Costa Mesa, California, is described in the Merck Index, compound No. 8199, Eleventh Edition. Its manufacture and formulation is described in U.S. Patent No. 4,211,771.

For the pediatric patient co-infected with the HIV-1 and HCV infections, a suitable HAART includes a NRTI+ a PI, e.g., Nelfinavir +a NNRTI, e.g., Efavirenz in combination with the dosages and dosage regimens for pegylated interferon alfa and ribavirin listed herein above. See also Tables I-IV herein below. A human growth hormone such as the polypeptide hormone, somatropin, of recombinant rDNA origin, available under the HUMATROPE tradename from Eli Lilly & Co., Indianapolis, IN 46285, may be administered to these pediatric patients in the dosage and administration schedule listed in the product information sheet in consultation with the attending clinician to reduce retardation of growth associated with pegylated interferon alfa treatment.

HAART is administered to the patient in association with pegylated interferon-alfa, that is, the pegylated interferon-alfa dose may be administered before, after or during the same period of time that the patient receives doses of HAART. A human growth hormone such as the polypeptide hormone, somatropin, of recombinant rDNA origin, available under the HUMATROPE tradename from Eli Lilly & Co., Indianapolis, IN 46285, may also be administered - in association with HAART and pegylated interferon alfa - to the pediatric patient having HIV-1 infection in the dosage and administration schedule listed in the product information sheet in consultation with the attending clinician.

In a preferred embodiment of the present invention, pegylated interferon alfa is administered to HIV-1 infected patients prior to initiation of HAART, and preferably about two to about four weeks prior to initiation of HAART. In another preferred embodiment of the present invention, administeration of pegylated interferon alfa is initiated concurrently, i. e., on the same day with the administeration of HAART. In another preferred embodiment of the present invention the pegylated interferon-alfa is administered after the HIV-1 infected patient has initiated HAART.

The goal of the anti-HIV-1 therapy of the present invention is to reduce the HIV-1-RNA viral load below the detectable limit. The "detectable limit of HIV-1-RNA" in the context of the present invention means that there are fewer than about 200 to fewer than about 50 copies of HIV-1-RNA per ml of plasma of the patient as measured by quantitative, multi-cycle reverse transcriptase PCR methodology. HIV-1-RNA is preferably measured in the present invention by the methodology of Amplicor -1 Monitor 1.5 (available from Roche Diagnsotics)or of Nuclisens HIV-1 QT - 1. This methodology is described by Schooley, RT, Antiviral Therapy(1997), 2 (Suppl. 4):59-70.

The doses and dosage regimen of the NRTIs, NNRTIs and PI; IL-2, IL-12 and pentafuside will be determined by attending clinician in view of the approved doses and dosage regimen in the package insert or as set forth in the protocol taking into consideration the age, sex and condition of the patient and the severity of the HIV-1 infection. For the pediatric patient infected with the H IV-1, or co-infected with the H IV-1 and HCV infections a suitable HAART includes a NRTI+ a PI, e.g., Nelfinavir +a NNRTI, e.g., Efavirenz in combination with the dosages and dosage regimens for pegylated interferon alfa and ribavirin listed herein above. See alsoTables I-IV hereinafter for dosages and dosage regimens.

The following clinical protocols may be used to administer the anti-HIV-1 therapy of the present invention. Many modifications of this clinical protocol will be obvious to the skilled clinician, and the following Study Designs should not be interpreted as limiting the scope of the method of this invention which is only limited by the claims listed hereinafter. See for example J. G. McHutchinson et al. (N. Engl. J. Med., 1998, 339:1485-1492), and G. L. Davis et al. (N. Engl. J. Med. 339:1493-1499).

## STUDY N0. 1

The study population will include male and female patients diagnosed with HIV-1 infection who are either treatment naive or treatment-experienced and will be included if they meet the following inclusion and exclusion criteria:

## Subject Inclusion Criteria:

- Subjects diagnosed with HIV-1 infection who are either treatment naive or treatment-experienced.
- HIV-RNA by Amplicor test, Version 1.5 of greater than 500 copies/mL.
- $CD_4^+$ count greater than 100 copies/ml, preferably greater than 200 cells/mL.
- Subjects in good physical health with clinically acceptable safety laboratory test results and ECG.
- The following laboratory parameters must be met:

  - Platelet count ≥75,00/mL
  - Hemoglobin •9 gm/dL (90 gm/L)
  - Absolute neutrophil count •1500/µL
  - Creatinine ∠ 1.5 times the upper limit of normal
  - SGOT/SGPT ≤5 x upper limit of normal
  - Bilirubin ≤2.5 x upper limit of normal

- A negative urine pregnancy test (females only)

Subjects must be willing and able to give written informed consent and be able to adhere to the schedule set forth in the protocol.

**Subject Exclusion Criteria**

- Females who are breast-feeding or pregnant or who are not using adequate birth control.
- Subject with allergy to E. coli proteins
- Subjects with a significant past medical/psychiatric history, specifically depression or dementia.

The subjects will be randomized to receive pegylated interferon alfa 2b, i.e., $PEG_{12000}$-interferon alfa 2b at doses between 0.5 and 4.5 micrograms per kilogram e.g. at doses of 0.5, 1.0, 1.5, 3.0 or 4.5 micrograms per kilogram by subcutaneous injection once a week. HAART may also be initiated before or concurrently with the administration of the pegylated interferon alfa 2b, i.e., $PEG_{12000}$-interferon alfa 2b,i.e., PEG- Intron which is available from Schering Corp, Kenilworth, NJ.

Overall Design and Plan of the Study:

The primary efficacy objective will be lowering of the HIV-I-RNA plasma levels by a factor of $\geq 0.5 \log_{10}$.
Plasma HIV-1-RNA/qPCR testing will be performed by a central laboratory. A positive HIV-1-RNA assay result will be required at Baseline; only patients positive for HIV-1-RNA will be eligible to participate.

**STUDY N0. 2**

**Study Objectives**

The objectives of this study are to investigate: (i) the antiviral activity of various-doses of PEG-Intron in treatment-experienced, HIV-1 infected subjects failing their current anti-retroviral( anti-HIV-1) regimen, (ii) the antiviral activity of PEG-Intron in subjects starting a new optimized HAART regimen after failing their previous regimen and (iii) the safety, tolerability and pharmacokinetics of PEG-Intron.

**Study Design**

This is a Phase II, randomized, double-blind, parallel group, placebo-controlled, multi-center, efficacy and safety study in treatment experienced HIV-1 infected subjects. Individuals who achieve $\geq 0.5 \log_{10}$ reduction in HIV RNA at Week 4 will continue their PEG-Intron regimen with the addition of new optimized HAART for an additional 24 weeks. All other patients will be discontinued but will be asked to return for follow-up viral load and lymphocyte subset studies at Week 16 and Week 28. Subjects who were randomized to the placebo arm will be offered compassionate use of PEG-Intron when the optimal dose of PEG-Intron has been determined.

**Duration of Study**

PEG-Intron will be administered subcutaneously once per week for up to 28 weeks. There will be a 4 week post-treatment follow-up period.

**Study Population**

The study population will include a total of 300 male and female subjects with documented HIV-1 infection who have been treated continuously with combination antiretroviral therapy for at least six months prior to study entry. As a general guideline, patients should be in reasonably good health with a Karnofsky Performance Scale score $\geq 70$. Patients must meet the Inclusion and Exclusion Criteria listed below.

**Subject Inclusion Criteria**

- Documentation of HIV-1 infection by any licensed ELISA test kit and confirmed by a second method (e.g., Western Blot); or by HIV culture, HIV p24 antigen, plasma HIV RNA or proviral DNA.
- Subjects $\geq 18$ years old who have been on a continuous HAART regimen for at least six months, prior to randomization; and on the current regimen for at least 6 weeks.

- HIV-RNA increase $\geq$ 3-fold (0.5 $\log_{10}$) from previous nadir in the last 6 months using the same assay; or a previous plasma HIV RNA level <500 copies/mL on current antiretroviral regimen.
- HIV-RNA $\geq$ 2,000 copies/mL using same assay, on 2 separate occasions (at lease 7 days apart) prior to starting study drug.
- $CD_4^+$ cell count $\geq$ 200 cells/mL.
- Absence of active systemic opportunistic infection.
- The following laboratory parameters must be met:

  - ◆ Platelet count $\geq$ 75,000/mm$^3$
  - ◆ Hemoglobin $\geq$ 9gm/dL
  - ◆ Absolute neutrophil count $\geq$ 1,500/mm$^3$
  - ◆ Creatinine $\geq$ 1.5 times the upper limit of normal
  - ◆ SGOT/SGPT $\geq$ 5 times the upper limit of normal

- A negative serum pregnancy test (females of childbearing potential only).
- Subjects must be willing and able to give written informed consent and be able to adhere to the schedule set forth in the protocol.

## Subject Exclusion Criteria

- Ongoing Intron or PEG-Intron therapy within the last 6 months.
- Females who are breast-feeding or pregnant or of childbearing potential and not using adequate birth control measures, e.g. intrauterine medroxyprogesterone acetate [Depro-Provera], surgical sterilization, oral contraception, barrier method [diaphragm + spermicide or condom], during the treatment period.
- Individuals currently participating in, or who have participated in, a clinical trial of an investigational drug in the previous month.
- Subjects with a history of hypersensitivity to Interferons.
- Subjects with a history of severe depression, dementia, or other major psychiatric illness.
- Any CNS abnormality that requires use of antiseizure medication. • Unwillingness or inability to change to new optimized HAART.
- Unwillingness to wait until HIV resistance profile is available before changing current antiretroviral regimen.
- Intercurrent illness, vaccinations, or use of immunodulators (within 2 weeks prior to randomization) that could influence the plasma HIV RNA level.
- Use of systemic corticosteroids (excluding anabolic agents), immunosuppressants, ribavirin, or cytotoxic agents (including hydroxyurea) within 2 weeks prior to randomization.

In addition, study drug will be stopped and subjects will be discontinued from the study in the following situations: Baseline evaluations outside the range permitted by the protocol; failure to achieve at least 0.5 $\log_{10}$ reduction in HIV RNA at Week 4; use of a medication excluded by the protocol; subject enrollment in another investigational drug trial at any time during the study; medically serious or life threatening adverse events; protocol defined virologic failure; pregnancy during the treatment phase; failure to comply with dosing (as defined by protocol); subject unwillingness to continue.

## Method of Treatment Assignment (Randomization)

Subjects who meet the criteria for entry will be randomly assigned to one of five treatment groups in a 1:1:1:1:1 ratio.

## Study Treatments

Subjects will be randomized to one of the 5 following treatment groups:

- PEG-Intron 0.5 μg/kg once weekly SC
- PEG-Intron 1 μg/kg once weekly SC
- PEG-Intron 1.5 μg/kg once weekly SC
- PEG-Intron 3 μg/kg once weekly SC

- PEG-Intron placebo once weekly SC

All injections of PEG-Intron to be used in this study will be prepared not more than 24 hours prior to administration (provided the dose is kept refrigerated). PEG-Intron is supplied by Schering Corp. , Kenilworth, NJ 07033 as a lyophilized powder, and should be prepared as follows:

From a vial/ampule of Sterile Water for Injection, withdraw 0.7 mL with a syringe (e.g.,1/2 inch, 27 gauge needle on a 1.0 mL syringe). Add the sterile water to the vial containing the PEG-Intron lyophilized powder and mix gently. Inspect vial for discoloration or particulate matter and discard if present. On Day 1 of each week, PEG-Intron will be injected subcutaneously with the injection sites being rotated as necessary. Patients will receive one injection per week of PEG-Intron or placebo for up to 52 weeks.

### Administration of Study Medication

- <u>PEG-Intron/Placebo -</u> Dosing will be calculated on a μg/kg basis using the Baseline visit weight and will be administered (SC) subcutaneously. Subjects will administer PEG-Intron on the same day of the week each week.

Subjects may receive acetaminophen (500-1000 mg) 30 minutes prior to receiving PEG-Intron.

### Primary Endpoints

The primary endpoint will be the average change in HIV RNA from Baseline to Week 4 of PEG-Intron therapy. Baseline HIV RNA will be the mean of 2 RNA values, one obtained 7-14 days prior to starting study drug and the other obtained immediately prior to the first dose of study drug.

### Secondary Endpoints

Secondary endpoints include the following: AUC for HIV RNA from Baseline to Week 4; change in $CD_4^+$ + absolute count and percent from Baseline to Week 4; change in markers of T cell activation from Baseline to Week 4. In addition, the following parameters will be studied: change in viral load (and AUC) at Weeks 12, and 24 after starting new optimized HAART; change in $CD_4^+$ absolute count and percent at 12 and 24 weeks after new optimized HAART; time to HIV RNA decline below limit of quantitation of assay after starting new HAART; and change in HIV resistance profile of the individual components of each subject's HAART regimen at 24 weeks after new optimized HAART or at the time of virologic failure (whichever occurs first).

### Laboratory Evaluations

A central laboratory will be used for clinical laboratory testing during the study. Baseline results from the central laboratory will be used as a basis for determining whether a clinically significant change has occurred in a safety variable after the first dose of study medication. If necessary, additional laboratory assessments needed for subject safety during the study should be performed by the central lab. If this is not possible, local labs are acceptable.

### <u>STUDY NO. 3</u>

### Study Objectives

The objectives of this rising multiple-dose, open-label study are:(1) to assess the safety and tolerability of single and multiple doses of PEG-Intron in chronically HIV-infected pediatric patients who may have resistance to ongoing oral antiretroviral therapy, using a rising dose study design; (2) to measure the single and multiple dose pharmacokinetics of PEG-Intron in chronically HIV-infected pediatric patients; and (3) to examine the effect of PEG-Intron on HIV viral pharmacodynamics (HIV-RNA, $CD_4^+$ counts) and immune parameters.

### Study Design

This is a Phase I, open-label, rising multiple-dose, uncontrolled, multi-center, safety, tolerability, pharmacokinetic and pharmacodynamic study, in chronically HIV-I infected pediatric subjects. This study is also designed to determine the dose limiting toxicity (DLT) and maximum tolerated dose (MTD) of PEG-Intron in children when given once weekly for 4 weeks.

## Duration of Study

Study treatment will be administered once per week for 4 weeks.

## Study Population

Male and/or female HIV-infected pediatric patients aged 3-16 years will be enrolled into this study. Patients must have chronic HIV infection for >1 year. Patients must be established on antiretroviral therapy with detectable (>500 copies/mL) serum HIV-RNA titers. No change in antiretroviral regimen will be allowed within 6 weeks of study entry or while receiving PEG-Intron. Patients must meet the Inclusion and Exclusion Criteria listed below.

## Subject Inclusion Criteria

Subjects should be generally in good physical health with clinically acceptable safety laboratory test results and normal ECG.

- Subjects between the ages of 3 and 16 years, with chronic HIV-I infection on a stable antiretroviral regimen for at least 6 weeks. Subjects must remain on their antiretroviral regimen for the duration of the study.
- HIV-RNA levels as determined by Amplicor of greater than 500 copies/mL.
- %$CD_4^+$ counts greater than 200 cells/mL (CDC Immunologic Category 1 or 2).
- Absence of active systemic opportunistic infection on physical examination.
- No history of systemic opportunistic infection or immunization within the preceding 2 months
- The following laboratory parameters must be met:

  ♦ Platelet count ≥75,000/μL (75 x $10^9$/L)
  ♦ Hemoglobin ≥9 gm/dL (90 gm/L)
  ♦ Absolute neutrophil count ≥1500/μL (0.15 x $10^9$/L)
  ♦ Creatinine <1.5 times the upper limit of normal
  ♦ SGOT/SGPT ≤5 x upper limit of normal
  ♦ Bilirubin ≤2.5 x upper limit of normal

- A negative serum pregnancy test (females only).
- Parents/guardians must provide written informed consent, children capable of doing so will also given written informed consent. All participants must be able to adhere to the schedule set forth in the protocol.

## Subject Exclusion Criteria

- Subjects with clinically significant disorders not related to their HIV disease.
- Progression of HIV-I to AIDS.
- Females who are breastfeeding or pregnant or who are not using adequate birth control.
- Individuals who have participated in a clinical trial of an investigational drug in the previous month.
- Subjects who are positive for hepatitis B surface antigen or hepatitis C antibody.
- Subjects with allergy to E. coli proteins.
- Subjects with a significant past medical/psychiatric history, specifically depression.
- Presence of Interferon neutralizing antibodies (these results may not be available during screening; patients will be considered for Inclusion without these results).
- Patients with clinical history of drug dependence.

## Method of Treatment Assignment (Randomization)

Patient numbers will be centrally assigned. Treatment allocation will not be randomized in this study.
Four cohorts of subjects will receive PEG-Intron at doses of 0.75, 1.5 3.0 or 4.5 μg/kg by subcutaneous injection once per week (QW) for 4 weeks.
The cohort of patients will be enrolled at the 0.75 μg/kg dose level. Subsequent cohorts of patients will be enrolled at successively higher doses according to the dose escalation guidelines below.

**Study Treatments**

- PEG- Intron :0.75, 1.5 or 3.0 or 4.5 µg/kg SC, QW.

    All injections of PEG-Intron to be used in this study will be prepared not more than 24 hours prior to administration (provided the dose is kept refrigerated). PEG-Intron is supplied as a lyophilized powder, and should be prepared as follows:

    From a vial of Sterile Water for Injection, withdraw 0.7 mL with a syringe (e.g.,5/8 inch, 25 gauge needle on a 1.0 mL syringe). Add the sterile water to the vial containing the PEG-Intron lyophilized powder and mix gently. Inspect for discoloration or particulate matter. If present, discard vial. 0.5 mL of this solution contains 50 µg, 100 µg, 150 µg or 300 µg of PEG- Intron depending on the vial used. On Day 1 of each week, PEG- Intron will be injected subcutaneously with the injection sites being rotated as necessary. Patients will receive one injection per week for 4 weeks.

    Dosing volumes will be calculated using the following formula:

$$\text{Volume = body-weight x dose/PEG-Intron concentration}$$

- For the 0.75 µg/kg dose, using a 50 µg vial size:

$$\text{Volume (mL) = body-weight (kg) x 0.75 µg/kg/100 µg/mL}$$

- For the 1.5 µg/kg dose, using a 100 µg vial size:

$$\text{Volume (mL) = body-weight (kg) x 1.5 µg/kg/200 µg/mL}$$

- For the 3.0 µg/ml dose, using a 150 µg vial size:

$$\text{Volume (mL) = body-weight (kg) x 3.0 µg/kg/300 µg/mL}$$

- For the 4.5 µg/ml dose, using a 300 µg vial size:

$$\text{Volume (mL) = body-weight (kg) x 4.5 µg/kg/300 µg/mL}$$

All doses of PEG- Intron must be administered at the clinic under the direction of the study staff.

During the first 48 hours of therapy, the study physician, physician coordinator or nursing staff should be easily accessible to the subject since adverse events are typically the most severe following the first injection. Flu-like symptoms, fever, chills, fatigue and malaise may occur in subjects two to eight hours after the initial dose of study medication. Initial reactions are generally mild to moderate in nature, and in most subjects tachyphylaxis of these symptoms will occur.

For patients who complete all Week 4 procedures, and who in the opinion of the Investigator might benefit from continued treatment, PEG-Intron treatment may be continued post-study in a continuation study. During compassionate treatment, there will be monitoring of safety, tolerability, virology and immunological parameters by the Principal Investigator.

**Primary Endpoints**

The following are primary endpoints:

- Safety laboratory tests, vital signs, ECGs and reported adverse events during 4 weeks of treatment.
- Serum HIV-RNA titer and $CD_4^+$ cell counts during 4 weeks of treatment.

- Single and multiple-dose serum PEG-lntron concentrations (Weeks 1 and 4).

The primary objective of this study is to describe the safety and tolerability of PEG- Intron based on data from the 4 weeks of treatment. Concentration data and derived pharmacokinetic parameters (AUC and Cmax) of PEG-Intron, and HIV-RNA titers will be summarized using descriptive statistics. In addition to the analyses specified below, listings of all data for each patient will be provided.

Pretreatment (Days -28, -2 and Day 1, 0 hour) HIV-RNA and $CD_4^+$ counts will be used as Baselines for each individual. Relative to mean Baseline value, evidence of Interferon response will be based on >0.5 log reduction in HIV-RNA.

Table I

| NUCLEOSIDE REVERSE TRANSCRIPTASE INHIBITORS (NRTI) DOSAGE & DOSAGE REGIMEN | |
|---|---|
| NRTI (Tradename, Marketer) | Usual adult dosage |
| Zidovudine, AZT (Retrovir - Glaxo Wellcome)* | 200 mg PO tid or 300 mg PO bid |
| Stavudine (Zerit - Bristol-Myers Squibb)* | 40 mg PO bid[1] |
| Didanosine (Videx - Bristol-Myers Squibb)* | 200 mg PO bid[2] |
| Lamivudine (Epivir - Glaxo Wellcome)* | 150 mg PO bid[3] |
| Zalcitabine (Hivid - Roche) | 0.75 mg PO tid |
| Zidovudine plus lamivudine (Combivir-Glaxo Wellcome) | 1 tablet PO bid[4] |
| Abacavir (Ziagen-Glaxo-Wellcome) | 200 or 400 mg PO tid |
| Adefovir dipivoxil (Prevon-Gilead Sciences) | 125 or 200 mg PO qd[5] |
| Lobucavir (BMS-180194-BMS) | 200 mg PO bid[6] |
| BCH-10652 (Biochem Pharma) | 400 mg PO, qid[7] |
| Emitricitabine ((-)-FTC-Triangle Pharmaceuticals) | 200 mg PO qd[8] |
| Beta-L-FD4 (B-L-D4C-Vion Pharmaceutical) | 0.2-25 mg/ky/day[9] |
| DAPD (Triangle Pharmaceuticals) | _____ [10] |
| Lodenosine (FddA-U.S. Bioscience) | 1.6-3.2 mg/Kg PO bid[11] |

Footnotes Table I

* Available in a liquid formulation.

[1] For patients less than 60 kg, 30 mg PO bid.

[2] With tablets; for patients < 60 kg. 125 mg PO bid; >60 kg. 200 mg PO bid;. With powder, dosage varies from 167 mg (< 60 kg) to 250 mg PO (< 60 kg) bid. Doses should be taken at least 30 minutes before meals or at least two hours afterward.

[3] For patients less than 50 kg. 2 mg/kg PO bid.

[4] Each tablet contains 300 mg of zidovudine and 150 mg of lamivudine.

[5] Available under an expanded access program - a NIH-sponsored Phase III Trial

[6] Phase II

[7] Phase I/II; see PharmaProjects, sections J5A & J5Z

[8] Phase II/III; see PharmaProjects, sections J5A & J5Z

[9] Preclinical; active in duck HBV model; see PharmaProjects, sections J5A & J5Z

[10] Preclinical; active po and IV; DAPD is a prodrug of another dioxolene purine, DXG. See PharmaProjects, sections J5A & J5Z

[11] Phase II, FddA has potential for once-a-day dosage

TABLE II

| NON-NUCLEOSIDE REVERSE TRANSCRIPTASE INHIBITORS (NNRTI) Dosage and Dosage Regimen | |
|---|---|
| NRTI (Tradename, Marketer) | Usual adult dosage and Dosage Regimen |
| Nevirapine (Viramune - Roxane) | 200 mg PO bid[1] |
| Delavirdine (Rescriptor - Pharmacia & Upjohn) | 400 mg PO tid |
| Efavirenz (Sustiva, Dupont) | 200 mg PO qid[2] |
| PNU-142721 (Pharmacia + Upjohn) | _____ [3] |

[1] For the first two weeks of treatment with nevirapine, to decrease the risk of rash, patients should take only one 200-mg tablet per day.

[2] Quadruple Therapy of efavirenz with indinavir + 2 NRTIs or Triple Therapy of efavirenz + AZT + lamivudine.

[3] Preclinical Phase; see PharmaProjects, sections J5A & J5Z

TABLE II   (continued)

| NON-NUCLEOSIDE REVERSE TRANSCRIPTASE INHIBITORS (NNRTI) Dosage and Dosage Regimen ||
| NRTI (Tradename, Marketer) | Usual adult dosage and Dosage Regimen |
| --- | --- |
| AG-1549 (Agouvon Pharmaceuticals) | _____ [4] |
| MKC-442 (Triangle Pharmaceuticals) | 750 mg PO bid[5] |
| (+)-Calanolide A (Med Chem Research) | 800 mg PO[6] |

[4] Phase I/II evaluating dose and comcomitant use with other anti-HIV-1 therapies; see PharmaProjects, sections J5A & J5Z.

[5] Triple Therapy of (a) MKC-442 with stavudine and either lamivudine or didanosine or (b) MKC-442 with nelfinavir (qv) and NRTIs.

[6] Phase I; see PharmaProjects, sections J5A & J5Z

TABLE III

| Protease Inhibitor (PI) Dosage and Dosage Regimen ||
| PI (Tradename, Marketer) | Dosage + Dosage Regimen |
| --- | --- |
| Saquinavir (Invirase - hard gel capsule- Roche) | 600 mg PO tid[1] |
| (Fortovase - soft gel capsule -Roche) | 1100 mg PO tid[1] |
| Ritonavir (Norvir - Abbott) | 600 mg PO bid[2] |
| Indinavir (Crixivan - Merck) | 800 mg PO qid[3] |
| Nelfinavir (Viracept - Agouron) | 750 mg PO tid[4] |
| Amprenavir (141W94, Glaxo) | 900 mg - 1200 mg PO bid[5] |
| Lasinavir (BMS-234475, BMS) | _____ [6] |
| DMP-450 (Triangle Pharmaceuticals) | _____ [7] |
| BMS-2322623 (BMS) | _____ [8] |
| ABT-378 (Abbott) | 60 mg PO bid[9] |

[1] With, or within two hours after, a full meal.

[2] With food. The liquid formulation has an unpleasant taste; the manufacturer suggests taking it with chocolate milk or a liquid nutritional supplement.

[3] With water, one hour before or two hours after a meal. Patients taking indinavir should drink at least 48 ounces (1.5 liter) of water daily.

[4] With food.

[5] Quadruple Combination Therapy of amprenavir with AZT + lamivudine + abacavir.

[6] Phase I/II; see PharmaProjects, sections J5A & J5Z.

[7] Phase II; see PharmaProjects, sections J5A & J5Z.

[8] Preclinical Studies; Prodrug esters of BMS 2322623 enhance oral absorption; see PharmaProjects, sections J5A& J5Z.

[9] Phase I Studies show ABT-378 to be 10X more potent than ritonavir; see PharmaProjects sections J5A & J5Z.

TABLE IV

| Other Anti-HIV-1 Drugs ||
| Drug (Trade Name, Marketer) | Usual Adult Dosage and Dosage Regimen |
| --- | --- |
| Hydroxyurea (Droxia, BMS) | 1000 mg PO qid[1] |
| Ribavirin( Rebetol, Schering-Plough) | 600mg-1200mg/day,PO |
| IL-2(Proleukin, Chiron Corp.) | 1 -20milliom IU/day,sc |
| IL-12(Roche) | 0.5-10 micrograms/kg/day, sc |
| Yissum Project No. 11607 (Yissum) | _____ [2] |

[1] Triple Therapy of hydroxyurea with 400 mg ddl + 500 mg AZT ; see PharmaProjects,section B3C1

[2] Preclinical; see PharmaProjects, sections J5A & J5Z.

**Claims**

1. The use of pegylated interferon-alfa for preparation of a medicament for treatment of HIV-1 infections in patients.

2. The use of claim 1 wherein the pegylated interferon-alfa-2b is administered in association with a therapeutically effective amount of at least one of ribavirin, IL-2, IL-12, and pentafuside alone or in combination with a therapeutically effective amount of an anti-HIV-1 drug therapy.

3. The use of an anti-HIV-1 drug therapy and pegylated interferon-alfa for preparation of a medicament for treatment of HIV-1 infections in patients.

4. The use of claim 2 or 3 wherein the anti-HIV-1 drug therapy is HAART.

5. The use of any preceding claim wherein the treatment comprises a therapeutically effective amount of pegylated interferon-alfa and a therapeutically effective amount of an anti-HIV-1 drug therapy sufficient to lower detectable HIV-1-RNA in such patients.

6. The use of any preceding claim wherein the patients are treatment-experienced patients or treatment-naive patients.

7. The use of any preceding claim wherein the patients are treatment-experienced or treatment-naïve pediatric patients.

8. The use of any preceding claim wherein the pegylated interferon-alfa is pegylated interferon alfa-2a or pegylated interferon alfa-2b.

9. The use of any preceding claim wherein the pegylated interferon-alfa is a pegylated interferon alfa-2b and wherein the treatment comprises an amount of pegylated interferon alfa-2b in the range of about 0.1 to about 9.0 micrograms per kilogram in a single dose QW or divided doses BIW, preferably in the range of about 0.5 to about 3.0 micrograms per kilogram in a single dose QW or divided doses BIW, and more preferably in the range of about 0.75 to about 1.5 micrograms per kilogram in a single dose QW or divided doses BIW.

10. The use of any preceding claim wherein wherein the pegylated interferon-alfa is a pegylated interferon alfa-2b and wherein the treatment comprises pegylated interferon alfa-2b in association with an anti-HIV-1 drug therapy, which preferably is HAART.

11. The use of any preceding claim wherein the pegylated interferon-alfa is a pegylated interferon alfa-2a and the treatment comprises an amount of pegylated interferon alfa-2a administered in the range of about 50 to about 500 micrograms in a single dose QW or divided doses BIW, preferably in the range of about 200 to about 250 micrograms in a single dose QW or divided doses BIW.

12. The use of any preceding claim, wherein the patient is co-infected with HCV.

13. The use of any preceding claim, wherein the treatment comprises pegylated interferon which is administered QW or BIW for at least about 2 to 4 weeks prior to administering a therapeutically effective amount of at least one of ribavirin, IL-2, IL-12, pentafuside alone or in combination with a therapeutically effective amount of an anti-HIV-1 drug therapy, and preferably, wherein the anti-HIV-1 drug therapy is HAART.

14. The use of any preceding claim, wherein the treatment comprises (1) about 0.1 to about 9.0 micrograms per kilogram of pegylated interferon-alfa-2b administered in a single dose QW or divided doses BIW in a first treatment time period of at least about two to about four weeks , followed by (2) a therapeutically effective amount of an anti HIV-1 drug therapy in association with about 0.1 to about 9.0 per micrograms per kilogram of pegylated interferon-alfa-2b administered as a single dose QW or divided doses BIW in a second treatment time period, sufficient to lower detectable HIV-1-RNA.

15. The use of claim 7, wherein the pegylated interferon-alfa is a pegylated interferon alfa-2b and wherein the treatment comprises an amount of pegylated interferon alfa-2b administered in the range of about 0.1 to about 9.0 micrograms per kilogram QW, preferably in the range of about 0.5 to about 4.5 micrograms per kilogram in single doses QW, or in divided doses BIW and more preferably, in the range of about 2.25 to about 2.6 micrograms per kilogram in single doses QW, or in divided doses BIW

16. The use of claim 7, wherein the pegylated interferon-alfa is a pegylated interferon alfa-2a and the treatment comprises an amount of pegylated interferon alfa-2a in the range of about 50 to about 500 micrograms in single doses QW, or in divided doses BIW. or preferably in the range of about 150 to about 190 micrograms in single doses QW, or in divided doses, BIW..

17. The use of claim 7, wherein the pediatric patient is co-infected with HCV, and the treatment further comprises ribavirin which is administered to the pediatric patient in association with the pegylated interferon-alfa and wherein the amount of ribavirin is in a range of about 8 to about 15 mg per kilogram per day, in divided doses.

18. The use of claim 7, wherein the treatment further comprises a human growth hormone in association with an anti-HIV-1 drug therapy and a pegylated interferon-alfa.

19. The use of claim 18, wherein the pediatric patient is co-infected with HCV and HIV-1 and the treatment further comprises ribavirin.

20. The use of claim 7, wherein the treatment further comprises a human growth hormone which is administered in association with a therapeutically effective amount of an anti-HIV-1 drug therapy and a therapeutically effective amount of pegylated interferon-alfa.and therapeutically effective amount of ribavirin.